# FASCICULE DE BREVET EUROPEEN

(11) **EP 2 435 180 B1**
(45) Date de publication et mention de la délivrance du brevet: **07.10.2015**
(21) Numéro de dépôt: 10728769.0
(22) Date de dépôt: 18.05.2010
(51) Int. Cl.: C07C 67/03, C07C 69/54, B01J 31/12

(54) **COMPOSITION COMPRENANT UN OXYDE DE DIALKYL ÉTAIN ET SON UTILISATION COMME CATALYSEUR DE TRANSESTÉRIFICATION POUR LA SYNTHÈSE D'ESTERS (MÉTH)ACRYLIQUES**
ZUSAMMENSETZUNG MIT DIALKYLZINNOXID UND IHRE VERWENDUNG ALS TRANSESTERIFIZIERUNGSKATALYSATOR ZUR SYNTHESE VON (METH)ACRYLESTERN
COMPOSITION INCLUDING DIALKYL TIN OXIDE AND USE THEREOF AS A TRANSESTERIFICATION CATALYST FOR THE SYNTHESIS OF (METH)ACRYLIC ESTERS

(30) Priorité: 26.05.2009 FR 0953433
(43) Date de publication de la demande: 04.04.2012
(73) Titulaire: Arkema France, 92700 Colombes (FR)
(72) Inventeur: PAUL, Jean-Michel, 57070 Metz (FR); TONNELIER, Boris, 57880 Guerting (FR); AUGUSTIN, Francis, 57260 Lindre-basse (FR)
(74) Mandataire: Bonnel, Claudine
(86) Numéro de dépôt international: PCT/FR2010/050949
(87) Numéro de publication internationale: WO 2010/136696

(56) Documents cités:
- US-A1- 2006 173 191
- US-B2- 7 078 560

## Description

La présente invention a trait à une composition comprenant un oxyde de dialkyl étain utilisable comme catalyseur de transestérification pour la synthèse d'esters (méth)acryliques. L'invention porte également sur un procédé de synthèse d'esters (méth)acryliques par transestérification en présence de cette composition.

Les dérivés organiques de l'étain, en particulier les oxydes de dialkyl étain, tel que l'oxyde de dibutyl étain (Bu₂Sn=O, dénommé ci-après DBTO) sont largement décrits dans la littérature comme catalyseurs de transestérification pour la synthèse d'esters (méth)acryliques.

Le DBTO se présente sous forme d'une poudre blanche insoluble dans l'eau, et pratiquement insoluble dans les alcools tels que le méthanol ou l'éthanol, ou les aminoalcools, dans les solvants hydrocarbonés, ainsi que dans les esters (méth)acryliques tels que l'acrylate de méthyle ou l'acrylate de diméthylaminoéthyle. Il se dissout lentement dans les solutions alcalines ; il est solubilisé dans les acides carboxyliques avec lesquels il forme des dérivés carboxylés.

Le DBTO sous forme de solide est largement utilisé dans des procédés de transestérification de type « batch ». Sa mise en oeuvre est relativement simple dans ce cas, bien qu'il nécessite cependant d'être manipulé avec soin, en raison de son état pulvérulent pouvant entrainer des risques d'inhalation d'un produit présentant une toxicité liée à celle de l'étain.

La mise en oeuvre du DBTO sous forme solide s'avère plus difficile dans le cas d'un procédé de transestérification en continu, ou lorsqu'il y a nécessité d'introduire le DBTO en continu. Des solutions existent, par exemple l'utilisation d'un système d'introduction de la poudre par vis sans fin, mais ces solutions sont très lourdes et ne dispensent pas des risques d'inhalation de particules fines de composé à base d'étain.

La Demanderesse a trouvé un moyen simple et original d'utiliser le DBTO sous forme d'une solution concentrée. De ce fait, il devient aisé de l'introduire en continu sous forme de solution à l'aide d'une simple pompe avec tous les avantages que cela représente en terme de facilité de mise en oeuvre et d'hygiène.

L'utilisation de dérivés organiques de l'étain, notamment le DBTO et ses homologues, pour la synthèse d'esters (méth)acryliques est largement décrite dans la littérature.

On peut citer la demande de brevet JP 56-104851 qui décrit l'utilisation d'un oxyde d'étain, en particulier le DBTO, comme catalyseur de transestérification pour la synthèse du méthacrylate de ter-butyl aminoéthyle à partir de méthacrylate de méthyle et de ter-butyl aminoéthanol.

Le brevet US 3,642,877 décrit l'utilisation du DBTO comme catalyseur pour la synthèse du méthacrylate de diméthylaminoéthyle par transestérification entre le méthacrylate de méthyle et le N,N diméthylaminoéthanol. Le DBTO est utilisé sous forme solide et il n'est introduit dans le milieu réactionnel qu'après avoir effectué une distillation azéotropique de l'eau avec le méthacrylate de méthyle afin d'éviter toute désactivation du DBTO par l'eau présente dans les réactifs.

La demande de brevet JP-A1-3-118,352 décrit un procédé de synthèse d'esters dialkyl aminoalkyle de l'acide (méth)acrylique par transestérification dans un solvant inerte à la réaction, tel que l'hexane, sous une pression de 1,5 à 3 atmosphères en présence de DBTO en poudre comme catalyseur.

Dans la demande de brevet US 2006/0173191, il a été proposé d'introduire le catalyseur de transestérification tel que le DBTO, sous forme de plusieurs charges dans le réacteur de réaction, le catalyseur étant introduit sous forme solide, ou en mélange avec du brut réactionnel prélevé dans le réacteur, ou encore sous forme de suspension dans le méthacrylate de méthyle. Dans ces conditions, il n'est pas suggéré de mettre en oeuvre le DBTO en solution concentrée homogène.

Dans la demande de brevet JP 2001-187763, l'acide (méth)acrylique correspondant à l'ester (méth)acrylique visé est utilisé pour solubiliser un oxyde de dialkyl étain avant d'être introduit sous forme de solution comme catalyseur de transestérification, ce qui permet d'éviter tout problème de reprécipitation ou de dépôt de solide lorsque la température réactionnelle diminue.

Le brevet US 7,078,560 revendique l'utilisation de dérivés organiques de l'étain sous forme de distannoxanes comme catalyseurs de transestérification dans la synthèse de (méth)acrylates d'aminoalcools, pour pallier le problème d'insolubilité du DBTO dans le N,N diméthylaminoéthanol et plus généralement dans les aminoalcools. Le distannoxane est obtenu par réaction d'un oxyde organique d'étain avec son halogénure d'étain correspondant. A titre d'exemple l'octabutyl tétrachlorodistannoxane est préparé à partir de DBTO et de dichlorure de dibutyl étain (Bu₂SnCl₂). Ce distannoxane peut être solubilisé à hauteur de 10% massique ou plus, dans le N,N diméthylaminoéthanol. Selon cette méthode, le DBTO, pour être solubilisé dans le N,N diméthylaminoéthanol doit être préalablement transformé en une autre entité chimique, à savoir un distannoxane, ce qui entraine une préparation complexe pour le catalyseur.

La Demanderesse a trouvé un moyen plus simple visant à mettre en solution de l'oxyde de dibutyl étain à des teneurs relativement concentrées, de façon à générer des solutions catalytiques aptes à être utilisées directement dans des réactions de transestérification.

De façon surprenante, alors que le DBTO est insoluble dans les alcools et les esters, il a été trouvé que l'oxyde de dibutyl étain se solubilisait dans un mélange alcool/ester(s) (méth)acrylique(s). C'est par exemple le cas pour un mélange d'alcool et de (méth)acrylate de méthyle ou d'éthyle pris seuls ou en présence de l'ester lourd issu de la réaction de transestérification entre le (méth)acrylate de méthyle ou d'éthyle, et l'alcool.

La présente invention a donc pour but de fournir une solution concentrée en oxyde de dialkyl étain, plus particulièrement en DBTO, homogène et stable au cours du temps à température ambiante, et répondant aux exigences d'hygiène et sécurité en matière de législation des ateliers industriels.

Le but de la présente invention est aussi de fournir une composition catalytique comprenant du DBTO ou un homologue, utilisable pour la synthèse d'esters (méth)acryliques par transestérification, préparée préalablement avec des composés purs pouvant être mis en oeuvre dans cette synthèse, présentant une facilité de mise en oeuvre dans un procédé de type continu, ou lorsque l'introduction du catalyseur en continu est nécessaire, et conduisant à une sélectivité améliorée par rapport au DBTO solide ou son homologue.

La présente invention a pour premier objet une composition sous forme de solution homogène pouvant être conservée à température ambiante sans donner lieu à de précipitation de solide, comprenant en poids, le total faisant 100% :
- de 5% à 75% d'un oxyde de dialkyl étain, la chaîne alkyle linéaire ou ramifiée, ayant de 1 à 8 atomes de carbone,
- de 10% à 80% d'un alcool R₁OH (I), R₁ pouvant être un radical alkyle linéaire ou ramifié, ou un radical aliphatique cyclique, aryle, alkyle-aryle ou aryle-alkyle, comportant de 4 à 40 atomes de carbone, ou un radical alkyle linéaire ou ramifié contenant au moins un hétéroatome et de 3 à 40 atomes de carbone, choisi parmi le butanol, le 2-éthyl hexanol, le décanol, le N, N diméthylaminoéthanol, le N, N diméthylaminopropanol, le N, N diéthylaminoéthanol ou le tertiobutylaminoéthanol,
- de 10% à 80% d'un (méth)acrylate d'alkyle léger (II), la chaîne alkyle linéaire ou ramifiée ayant de 1 à 4 atomes de carbone, choisi parmi le (méth)acrylate de méthyle ou le (méth)acrylate d'éthyle,
- de 0 à 80% d'un ester (méth)acrylique (III) comprenant un radical pouvant être un radical alkyle linéaire ou ramifié, ou un radical aliphatique cyclique, aryle, alkyle-aryle ou aryle-alkyle, comportant de 4 à 40 atomes de carbone, ou un radical alkyle linéaire ou ramifié contenant au moins un hétéroatome et de 3 à 40 atomes de carbone, choisi parmi le (méth)acrylate de butyle, le (méth)acrylate de 2-éthyl hexyle, l'acrylate de diméthylaminoéthyle, l'acrylate de diméthylaminopropyle, l'acrylate de diéthylaminoéthyle, l'acrylate de tertiobutylaminoéthyle ou le méthacrylate de diméthylaminoéthyle.

Par le terme (méth)acrylate, on entend les dérivés de l'acide acrylique ou de l'acide méthacrylique.

Comme oxyde de dialkyl étain, on peut utiliser l'oxyde de diméthyl étain, l'oxyde de méthyl éthyl étain, l'oxyde de diéthyl étain, l'oxyde de dipropyl étain, l'oxyde de n-dibutyl étain, ou l'oxyde de dioctyl étain. On utilise de préférence un oxyde de dialkyl étain de chaîne alkyle linéaire ayant de 1 à 4 atomes de carbone, plus particulièrement l'oxyde de n-dibutyl étain (DBTO).

Avantageusement, la composition comprend de 50% à 70% en poids d'oxyde de dialkyl étain, de préférence de 55% à 65%. Dans ces gammes de concentration, les solutions obtenues sont stables à température ambiante, c'est à dire qu'elles ne donnent pas lieu à de précipitation de solide.

Comme alcools (I) utilisables, de préférence, on utilise un aminoalcool tel que le N, N diméthylaminoéthanol.

Comme (méth)acrylate d'alkyle léger (II), on peut utiliser le (méth)acrylate de méthyle ou le (méth)acrylate d'éthyle.

De façon optionnelle, la composition comprend un ester (méth)acrylique (III) qui est choisi parmi le (méth)acrylate de butyle, le (méth)acrylate de 2-éthyl hexyle, l'acrylate de diméthylaminoéthyle (ADAME), l'acrylate de diméthylaminopropyle, l'acrylate de diéthylaminoéthyle, l'acrylate de tertiobutylaminoéthyle, le méthacrylate de diméthylaminoéthyle (MADAME). De préférence on utilise l'acrylate de diméthylaminoéthyle (ADAME).

Les teneurs respectives en alcool, en (méth)acrylate d'alkyle léger et en ester (méth)acrylique dépendent de la nature des produits engagés pour effectuer la mise en solution de l'oxyde de dialkyle étain. De préférence, la composition telle que définie selon le premier objet de l'invention comprend en poids, le total faisant 100% :
- de 50% à 70% d'un oxyde de dialkyl étain, de préférence de DBTO,
- de 10% à 40% d'un alcool R₁OH (I),
- de 10% à 40% d'un (méth)acrylate d'alkyle léger (II)
- de 0 à 40% d'un ester (méth)acrylique (III).

Une composition préférée selon l'invention comprend en poids, le total faisant 100% :
- de 55% à 65% de DBTO,
- de 10% à 20% d'un alcool R₁OH (I),
- de 10% à 20% d'un (méth)acrylate d'alkyle léger (II),
- de 0 à 20% d'un ester (méth)acrylique (III).

L'alcool (I) est de préférence le N,N diméthylaminoéthanol, et l'ester (méth)acrylique (III), l'ADAME ou le MADAME.

La composition selon l'invention est obtenue en chauffant, sous agitation, à une température comprise entre 80°C et 130°C et jusqu'à mise en solution, un mélange constitué d'oxyde de dialkyl étain, notamment de DBTO, d'un alcool R₁OH (I), d'un (méth)acrylate d'alkyle léger (II) et aussi préférentiellement, pour accélérer la mise en solution, d'un ester (méth)acrylique (III).

Plus précisément, la composition selon l'invention est préparée en introduisant le DBTO, ou son homologue, en une ou plusieurs fois et sous forte agitation, à température ambiante ou à chaud, sous pression atmosphérique ou sous légère dépression dans le mélange alcool R₁OH/(méth)acrylate d'alkyle léger avec ou sans ester (méth)acrylique, puis en chauffant le mélange à une température comprise entre 80°C et 130°C jusqu'à l'obtention d'une solution limpide. Lorsque la mise en solution est effectuée, le chauffage peut être prolongé de quelques heures avant refroidissement à température ambiante.

La solution obtenue, homogène, est conservée à température ambiante, de préférence à l'abri de la lumière, sans donner lieu à de précipitation de solide.

Il est préférable de protéger les dérivés méth)acryliques présents dans la composition selon l'invention contre la polymérisation à l'aide d'inhibiteurs de polymérisation dont l'action peut être favorisée à l'aide d'un bullage d'air dans le mélange, notamment d'air appauvri à 8% vol d'O₂.

Comme inhibiteurs de polymérisation, on peut utiliser de manière non limitative, l'hydroquinone, l'éther méthylique d'hydroquinone, le 2,6-diterbutyl 4-méthyl phénol (BHT), la phénothiazine, le 2,2,6,6-tétraméthyl-1-piperidinyloxy (TEMPO) ou ses dérivés, la paraphénylène diamine, seuls ou en mélange, à raison de 100 à 5000 ppm, de préférence de 500 à 3000 ppm pour chaque inhibiteur introduit dans le mélange.

La composition selon l'invention est avantageusement utilisée comme composition catalytique pour la synthèse d'esters (méth)acryliques par transestérification d'un (méth)acrylate d'alkyle léger avec un alcool.

Le choix des produits utilisés pour effectuer la mise en solution du DBTO sera de préférence adapté à la nature de l'ester (méth)acrylique qui sera synthétisé en utilisant cette solution comme catalyseur. Dans tous les cas, il s'agit de produits « purs », et non de phases prélevées dans un mélange brut réactionnel.

Ainsi, il est préférable d'utiliser l'alcool qui sera mis en oeuvre pour la transestérification. Par exemple, lorsqu'on prépare une solution de DBTO pour catalyser la synthèse de l'ADAME à partir d'acrylate de méthyle (AM) ou d'acrylate d'éthyle (AE) et de N,N diméthylaminoéthyle, on utilisera le N,N diméthylaminoéthanol en guise d'alcool R₁OH (I).

De même, on utilisera préférentiellement pour (II) et (III), respectivement le (méth)acrylate d'alkyle léger qui sera mis en oeuvre pour la synthèse de l'ester (méth)acrylique par transestérification, et l'ester ainsi produit.

Dans le cas précédent, on utilisera pour (II) l'acrylate de méthyle ou l'acrylate d'éthyle, et pour (III) l'acrylate de N ,N diméthylaminoéthyle (ADAME).

C'est ainsi qu'il a pu être préparé des solutions à 60 - 70% en poids de DBTO en chauffant du DBTO dans un mélange de N,N diméthylaminoéthanol, d'acrylate de méthyle et d'acrylate de N,N diméthylaminoéthyle, lesdites solutions étant ensuite utilisées directement pour fabriquer l'ADAME dans un procédé en continu.

L'invention porte aussi sur l'utilisation d'une composition comprenant en poids, le total faisant 100% :
- de 55% à 65% d'oxyde de dibutyl étain
- de 10% à 20% de N,N diméthylaminoéthanol,
- de 10% à 20% de (méth)acrylate de méthyle ou de (méth)acrylate d'éthyle,
- de 0 à 20% de (méth)acrylate de diméthylaminoéthyle,
comme catalyseur de transestérification pour la synthèse de (méth)acrylate de diméthyaminoéthyle à partir de N,N diméthylaminoéthanol et de (méth)acrylate de méthyle ou de (méth)acrylate d'éthyle.

Dans le cas où la solution de DBTO servira à catalyser la synthèse de l'acrylate de butyle par transestérification entre l'AE et le butanol, on choisira respectivement, pour (I) le butanol, pour (II) l'acrylate d'éthyle, pour (III) l'acrylate de butyle.

La présente invention a aussi pour objet un procédé de synthèse en continu d'esters (méth)acryliques de formule (III) : 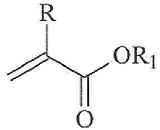 dans laquelle R est un atome d'hydrogène ou un groupement méthyle, et R₁ est un radical alkyle linéaire ou ramifié, ou un radical aliphatique cyclique, aryle, alkyle-aryle ou aryle-alkyle, comportant de 4 à 40 atomes de carbone, ou un radical alkyle linéaire ou ramifié contenant au moins un hétéroatome et de 3 à 40 atomes de carbone,
par réaction de transestérification d'un (méth)acrylate d'alkyle léger de formule (II) : 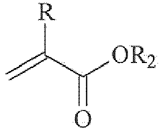 dans laquelle R a la signification précitée et R₂ une chaîne alkyle linéaire ou ramifiée ayant de 1 à 4 atomes de carbone
avec un alcool de formule (I) :

R₁-OH

dans laquelle R₁ a la signification précitée,
en présence d'un catalyseur de transestérification, caractérisé en ce que le catalyseur est une composition sous forme de solution homogène pouvant être conservée à température ambiante sans donner lieu à de précipitation de solide, telle que décrite précédemment.

De préférence, la composition catalytique est à base de DBTO.

La quantité de composition catalytique à utiliser est calculée à partir du nombre de moles de DBTO à mettre en oeuvre par mole d'alcool R₁OH mis en oeuvre dans la réaction de transestérification.

La quantité molaire de DBTO est comprise entre 0,001 et 0,02 mole/ mole d'alcool R₁OH mis en oeuvre dans la réaction de transestérification, de préférence entre 0,005 et 0,015 mole/ mole d'alcool R₁OH.

Le procédé selon l'invention est utilisé notamment pour préparer l'acrylate de diméthylaminoéthyle (ADAME), l'acrylate de diméthylaminopropyle, l'acrylate de diéthylaminoéthyle, l'acrylate de tertiobutylaminoéthyle, le méthacrylate de diméthylaminoéthyle (MADAME), de préférence l'ADAME.

De préférence, le (méth)acrylate d'alkyle léger (II) est l'acrylate de méthyle ou l'acrylate d'éthyle.

Au cours de la synthèse est généré du méthanol ou de l'éthanol, qui est éliminé sous forme d'un azéotrope avec l'acrylate de méthyle ou l'acrylate d'éthyle (II) respectivement.

Dans le procédé selon l'invention, on choisit de préférence un rapport molaire (méth)acrylate de méthyle ou d'éthyle (II) sur alcool (I) compris entre 2 et 5, de préférence entre 2 et 4.

La température de réaction est généralement comprise entre 80°C et 130°C et la pression est généralement maintenue entre 2 kPa et la pression atmosphérique. De préférence, la température de réaction est comprise entre 90°C et 110°C et la pression est de l'ordre de 50 kPa à la pression atmosphérique.

La réaction est généralement effectuée en présence d'au moins un inhibiteur de polymérisation. Comme inhibiteur de polymérisation, on utilise la phénothiazine, l'hydroquinone, l'éther monométhylique d'hydroquinone, le diterbutyl para-crésol (BHT), la paraphénylène diamine, le TEMPO (2,2,6,6-tétraméthyl-1-piperidinyloxy), le di-tertiobutylcatéchol, ou les dérivés du TEMPO, seuls ou en mélange, à raison de 100 à 5000 ppm par rapport à la charge initiale, de préférence entre 500 et 3000 ppm.

Les réactifs (I) et (II) contenant éventuellement les inhibiteurs de polymérisation sont introduits en continu, en mélange ou séparément à l'aide de pompe(s). La composition catalytique est introduite séparément à l'aide d'une pompe, à température ambiante ou à chaud à une température comprise entre la tempéraure ambiante et 80°C.

La réaction peut être effectuée dans un réacteur agité mécaniquement ou par circulation forcée. Le mélange réactionnel est chauffé à l'aide d'une double enveloppe ou par circulation forcée au travers d'un échangeur extérieur. Le réacteur est surmonté d'une colonne à distiller équipée d'un condenseur de tête, d'une tête de reflux, d'un séparateur à vide avec recette et piège.

Le procédé selon l'invention est particulièrement avantageux puisqu'il n'y a pas formation de précipité solide susceptible de boucher les conduites au cours de la réaction, et conduit à des sélectivités améliorées par rapport à celles obtenues avec du DBTO solide.

Les exemples suivants illustrent la présente invention sans toutefois en limiter la portée, Les pourcentages sont exprimés en pourcentages en poids.

### EXEMPLES

Les abréviations suivantes sont utilisées :
AM : acrylate de méthyle
AE : acrylate d'éthyle
ABU : acrylate de butyle
MAM : méthacrylate de méthyle
AOH : N,N diméthylaminoéthanol
ADAME : acrylate de N,N diméthylaminoéthyle
MADAME : méthacrylate de N,N diméthylaminoéthyle
PTZ : phénothiazine
EMHQ : éther méthylique d'hydroquinone
BHT : 2,6-diterbutyl 4-méthyl phénol

### Exemple 1

Dans un réacteur en verre agité mécaniquement et équipé d'une double enveloppe, on charge :
- 125g de N,N diméthylaminoéthanol (AOH)
- 145g d'acrylate de méthyle (AM)
- 125g d'acrylate de N,N diméthylaminoéthyle (ADAME)
- 600g de DBTO
- 3g de PTZ
- 2g de EMHQ

Le mélange est ensuite chauffé sous agitation par circulation d'huile thermostatée à 110°C dans la double enveloppe.

Après 1h30, le milieu est parfaitement limpide et homogène. On laisse 30 min de plus sous agitation. puis on refroidit la solution. Celle-ci est conservée à température ambiante à l'abri de la lumière avant utilisation comme catalyseur. Il n'y a pas de précipitation de solide. Cette solution a pu être utilisée pendant plusieurs semaines sans observer de phénomène de désactivation.

### Exemple 2

On prépare dans les mêmes conditions que celles de l'exemple 1 une solution comprenant :
- 125g de N,N diméthylaminoéthanol (AOH)
- 145g de méthacrylate de méthyle (MAM)
- 125g de méthacrylate de N,N diméthylaminoéthyle (MADAME)
- 600g de DBTO
- 3g de PTZ
- 2g de EMHQ

### Exemple 3

On prépare dans les mêmes conditions que celles de l'exemple 1 une solution comprenant :
- 125g de n-butanol
- 145g d'acrylate d'éthyle (AE)
- 125g d'acrylate de butyle (ABU)
- 600g de DBTO
- 3g de PTZ
- 2g de EMHQ

### Exemple 4

L'exemple 1 est repris avec :
- 125g de N,N diméthylaminoéthanol (AOH)
- 125g d'acrylate de méthyle (AM)
- 280g de DBTO
- 1,6 g de PTZ
- 1,1 g de EMHQ

Le mélange est chauffé pendant 4h à 100°C jusqu'à obtention d'une solution limpide. On laisse 30 min de plus sous agitation. puis on refroidit la solution. Celle-ci est conservée à température ambiante à l'abri de la lumière.

### Exemple 5 : Synthèse ADAME à partir d'AM

La solution catalytique de l'exemple 1 est utilisée pour préparer de l'ADAME par transestérification entre l'AM et AOH.

La réaction est effectuée dans un réacteur en verre agité, chauffé par circulation d'huile thermostatée dans une double enveloppe, surmonté d'une colonne à distiller munie d'un condenseur, avec tête de reflux, séparateur à vide, recette et piège.

Le réacteur est chargé avec de l'AM (432,7g), de l'AOH (279,9g), les inhibiteurs de polymérisation (PTZ 4000 ppm - BHT 2000 ppm) et la solution catalytique préparée à l'exemple 1 (13,05g, soit 0,01 mole DBTO/mole AOH mis en oeuvre pour la réaction).
Rapport molaire AM / AOH : 1,6 / 1

Le méthanol formé est distillé sous forme d'un azéotrope AM/méthanol au fur et à mesure de sa formation afin de déplacer l'équilibre réactionnel.

La réaction est effectuée en ajustant la pression afin de ne pas dépasser 110°C dans le réacteur.

Le brut est ensuite distillé sous une pression de 13 kPa à 8 kPa.

Quatre opérations successsives sont effectuées en chargeant l'AM et l'AOH sur le résidu de distillation contenant les inhibiteurs et le catalyseur.

| | OP n°1 | OP n°2 | OP n°3 | OP n°4 |
|---|---|---|---|---|
| Conversion AOH, % | 84,5 | 90 | 88,2 | 86,6 |
| Durée de réaction, h | 3 | 3,5 | 4 | 4 |

### Exemple 6 : Synthèse ABU à partir d'AE

On utilise l'appareillage décrit à l'exemple 5.

Le réacteur est chargé avec de l'AE (494,2g), du butanol (228,6g), les inhibiteurs (PTZ 4000 ppm - BHT 2000 ppm) et la solution catalytique de l'exemple 3 (12,8g, soit 0,01 mole DBTO/mole butanol mis en oeuvre pour la réaction).
Rapport molaire AE / butanol : 1,6 / 1

L'éthanol formé est distillé sous forme d'un azéotrope AE/éthanol au fur et à mesure de sa formation afin de déplacer l'équilibre réactionnel.

La réaction est effectuée en ajustant la pression afin de ne pas dépasser 110°C dans le réacteur.

Le brut est ensuite distillé sous une pression de 13 kPa à 8 kPa.
Durée de réaction : 4h30
Conversion du butanol : 99,3 %
Sélectivité en ABU : 99,5 %

### Exemple 7 : Synthèse MADAME à partir de MAM

On utilise l'appareillage décrit à l'exemple 5.

Le réacteur est chargé avec du MAM (581,2g), de l'AOH (258,6g), les inhibiteurs (PTZ 4000 ppm - BHT 2000 ppm) et la solution catalytique de l'exemple 2 (12,1g, soit 0,01 mole DBTO/mole AOH).
Rapport molaire MAM / AOH : 1,6 / 1

Le méthanol formé est distillé sous forme d'un azéotrope MAM/méthanol au fur et à mesure de sa formation afin de déplacer l'équilibre réactionnel.

La réaction est effectuée en ajustant la pression afin de ne pas dépasser 110°C dans le réacteur.

Le brut est ensuite distillé sous une pression de13 kPa à 8 kPa.
Durée de réaction : 5h30
Conversion de AOH : 99,7 %
Sélectivité en MADAME : 95,5 %

### Exemple 8 : Synthèse ADAME ex AM en continu

L'appareillage est similaire à celui utilisé pour les essais 5 à 7.

Le mélange de réactifs (AM, AOH, inhibiteurs) est introduit en continu dans le réacteur à l'aide d'une pompe à un débit de 156,7 g/h.

La solution catalytique de l'exemple 1 est introduite par pompe à un débit de 3,1 g/h.

Le méthanol formé est distillé sous forme d'un azéotrope AM/méthanol au fur et à mesure de sa formation afin de déplacer l'équilibre réactionnel.
Rapport molaire AM / AOH : 1,4 / 1
Catalyseur : 0,01 mole DBTO / mole AOH
Temps de passage dans le réacteur : 6h
Inhibiteurs : PTZ 3000 ppm / BHT 1000 ppm
T° : 95°C
Durée de l'essai : 72h

Le brut réactionnel est ensuite distillé. Le résidu contenant le catalyseur est recyclé en continu à l'essai suivant.

Les fractions légères de distillation sont recyclées à l'essai suivant avec un apport d'AM et d'AOH frais.

90% du résidu contenant le catalyseur est recyclé avec un appoint de solution catalytique fraîche.

Trois essais ont été réalisés dans ces conditions :

| | Essai 1 | Essai 2 | Essai 3 |
|---|---|---|---|
| Durée de l'essai, h | 72 | 47 | 24 |
| Conversion de AOH, % | 68,6 | 70,2 | 72,5 |
| Composition du brut en sortie du réacteur, % | | | |
| MeOH | 0,3 | 0,3 | 0,2 |
| AM | 13,7 | 12,0 | 11,2 |
| AOH | 18,2 | 15,1 | 14,3 |
| ADAME | 60,8 | 60,5 | 60,2 |

### Exemple 9 (comparatif)

On reproduit la synthèse de l'exemple 5 en utilisant du DBTO solide. Le rapport molaire DBTO/AOH est égal à 0,01 dans les 2 exemples.

| | Exemple 9 DBTO solide 7,7g pour 279,9 g AOH | Exemple 5 (OP n°1) DBTO en solution à 60% 12,8g de solution soit 7,7 g de DBTO pour 279,9 g AOH |
|---|---|---|
| Durée de réaction, h | 4 | 3 |
| Conversion AOH, % | 83,2 | 84,5 |
| Sélectivité ADAME, % | 92,7 | 94,9 |

## Revendications

1. Composition sous forme de solution homogène pouvant être conservée à température ambiante sans donner lieu à de précipitation de solide, comprenant en poids, le total faisant 100% :
- de 5% à 75% d'un oxyde de dialkyl étain, la chaîne alkyle linéaire ou ramifiée, ayant de 1 à 8 atomes de carbone,
- de 10% à 80% d'un alcool R₁OH (I), choisi parmi le butanol, le 2-éthyl hexanol, le décanol, le N, N diméthylaminoéthanol, le N, N diméthylaminopropanol, le N, N diéthylaminoéthanol ou le tertiobutylaminoéthanol,
- de 10% à 80% d'un (méth)acrylate d'alkyle léger (II), choisi parmi le (méth)acrylate de méthyle ou le (méth)acrylate d'éthyle,
- de 0 à 80% d'un ester (méth)acrylique (III) choisi parmi le (méth)acrylate de butyle, le (méth)acrylate de 2-éthyl hexyle, l'acrylate de diméthylaminoéthyle, l'acrylate de diméthylaminopropyle, l'acrylate de diéthylaminoéthyle, l'acrylate de tertiobutylaminoéthyle ou le méthacrylate de diméthylaminoéthyle.

2. Composition selon la revendication 1 comprenant :
- de 50% à 70% d'un oxyde de dialkyl étain,
- de 10% à 40% d'un alcool R₁OH (I),
- de 10% à 40% d'un (méth)acrylate d'alkyle léger (II),
- de 0 à 40% d'un ester (méth)acrylique (III).

3. Composition selon la revendication 1 ou 2 **caractérisée en ce que** l'oxyde de dialkyl étain est l'oxyde de dibutyl étain (DBTO).

4. Utilisation de la composition selon l'une quelconque des revendications précédentes comme catalyseur de transestérification pour la synthèse d'esters (méth)acryliques par transestérification d'un (méth)acrylate d'alkyle léger avec un alcool.

5. Utilisation selon la revendication 4 **caractérisée en ce que** les composés (I) et (II) présents dans la composition sont respectivement l'alcool et le (méth)acrylate d'alkyle léger mis en oeuvre dans la réaction de transestérification, et le composé (III) est l'ester (méth)acrylique produit par cette réaction.

6. Utilisation selon la revendication 5 d'une composition comprenant en poids, le total faisant 100% :
- de 55% à 65% d'oxyde de dibutyl étain
- de 10% à 20% de N,N diméthylaminoéthanol,
- de 10% à 20% de (méth)acrylate de méthyle ou de (méth)acrylate d'éthyle,
- de 0 à 20% de (méth)acrylate de diméthylaminoéthyle,
comme catalyseur de transestérification pour la synthèse de (méth)acrylate de diméthyaminoéthyle à partir de N,N diméthylaminoéthanol et de (méth)acrylate de méthyle ou de (méth)acrylate d'éthyle.

7. Procédé de synthèse en continu d'esters (méth)acryliques de formule (III) : 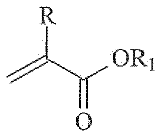 dans laquelle R est un atome d'hydrogène ou un groupement méthyle, et R₁ est un radical alkyle linéaire ou ramifié, ou un radical aliphatique cyclique, aryle, alkyle-aryle ou aryle-alkyle, comportant de 4 à 40 atomes de carbone, ou un radical alkyle linéaire ou ramifié contenant au moins un hétéroatome et de 3 à 40 atomes de carbone,
par réaction de transestérification d'un (méth)acrylate d'alkyle léger de formule (II) : 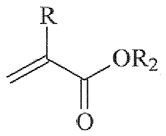 dans laquelle R a la signification précitée et R₂ une chaîne alkyle linéaire ou ramifiée ayant de 1 à 4 atomes de carbone
avec un alcool de formule (I) :
R₁-OH
dans laquelle R₁ a la signification précitée,
en présence d'un catalyseur de transestérification, **caractérisé en ce que** le catalyseur est une composition sous forme de solution homogène pouvant être conservée à température ambiante sans donner lieu à de précipitation de solide selon l'une quelconque des revendications 1 à 3.

8. Procédé selon la revendication 7 **caractérisé en ce que** les composés (I) et (II) présents dans la composition sont respectivement l'alcool et le (méth)acrylate d'alkyle léger mis en oeuvre dans la réaction de transestérification, et le composé (III) est l'ester (méth)acrylique produit par cette réaction.

## Patentansprüche

1. Zusammensetzung in Form einer homogenen Lösung, die bei Raumtemperatur aufbewahrt werden kann, ohne dass es zu einer Abscheidung von Feststoffen kommt, wobei sie nach Gewicht Folgendes umfasst, wobei die Summe 100 % ergibt:
- 5 % bis 75 % eines Dialkylzinnoxids, wobei die unverzweigte oder verzweigte Alkylkette 1 bis 8 Kohlenstoffatome aufweist,
- 10 % bis 80 % eines Alkohols R₁OH (I), der aus Butanol, 2-Ethylhexanol, Decanol, N,N-Dimethylaminoethanol, N,N-Dimethylaminopropanol, N,N-Diethylaminoethanol oder tert.-Butylaminoethanol ausgewählt ist,
- 10 % bis 80 % eines kurzkettigen Alkyl(meth)acrylats (II), das aus Methyl(meth)acrylat und Ethyl(meth)acrylat ausgewählt ist,
- 0 bis 80 % eines (Meth)acrylsäureesters (III), der aus Butyl(meth)acrylat, 2-Ethylhexyl(meth)acrylat, Dimethylaminoethylacrylat, Dimethylaminopropylacrylat, Diethylaminoethylacrylat, tert.-Butylaminoethylacrylat oder Dimethylaminoethylmethacrylat ausgewählt ist.

2. Zusammensetzung nach Anspruch 1, die Folgendes umfasst:
- 50 % bis 70 % eines Dialkylzinnoxids,
- 10 % bis 40 % eines Alkohols R₁OH (I),
- 10 % bis 40 % kurzkettigen Alkyl(meth)acrylats (II),
- 0 bis 40 % eines (Meth)acrylsäureester (III).

3. Zusammensetzung nach Anspruch 1 oder 2, **dadurch gekennzeichnet, dass** es sich bei dem Dialkylzinnoxid um Dibutylzinnoxid (DBTO) handelt.

4. Verwendung der Zusammensetzung nach einem beliebigen der vorhergehenden Ansprüche als Umesterungskatalysator für die Synthese von (Meth)acrylsäureestern durch Umesterung eines kurzkettigen Alkyl(meth)acrylats mit einem Alkohol.

5. Verwendung nach Anspruch 4, **dadurch gekennzeichnet, dass** es sich bei den Verbindungen (I) und (II), die in der Zusammensetzung vorliegen, um den Alkohol beziehungsweise um das kurzkettige Alkyl(meth)acrylat handelt, welche bei der Umesterungsreaktion eingesetzt werden, und bei der Verbindung (III) um den (Meth)acrylsäureester, welcher bei dieser Reaktion entsteht.

6. Verwendung nach Anspruch 5 einer Zusammensetzung, die nach Gewicht Folgendes umfasst, wobei die Summe 100 % ergibt:
- 55 % bis 65 % an Dibutylzinnoxid,
- 10 % bis 20 % an N,N-Dimethylaminoethanol,
- 10 % bis 20 % an Methyl(meth)acrylat oder an Ethyl(meth)acrylat,
- 0 bis 20 % an Dimethylaminoethyl(meth)acrylat, als Umesterungskatalysator für die Synthese von Dimethylaminoethyl(meth)acrylat ausgehend von N,N-Dimethylaminoethanol und von Methyl(meth)acrylat oder von Ethyl(meth)acrylat.

7. Verfahren zur kontinuierlichen Synthese von (Meth)acrylsäureestern der Formel (III): 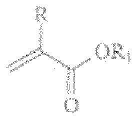 wobei R ein Wasserstoffatom oder eine Methylgruppe ist, und R₁ für einen unverzweigten oder verzweigten Alkylrest oder für einen zyklischen aliphatischen Rest, einen Aryl-, Alkylaryl- oder Arylalkylrest mit 4 bis 40 Kohlenstoffatomen, oder für einen unverzweigten oder verzweigten Alkylrest mit mindestens einem Heteroatom und 3 bis 40 Kohlenstoffatomen steht,
indem ein kurzkettiges Alkyl(meth)acrylat der Formel (II) : 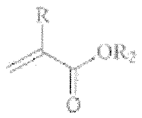 wobei R die vorstehend genannte Bedeutung hat und R₂ für eine unverzweigte oder verzweigte Alkylkette mit 1 bis 4 Kohlenstoffatomen steht,
mit einem Alkohol der Formel (I) umgeestert wird:
R₁-OH
wobei R₁ die vorstehend genannte Bedeutung hat,
in Gegenwart eines Umesterungskatalysators, **dadurch gekennzeichnet, dass** es sich bei dem Katalysator um eine Zusammensetzung in Form einer homogenen Lösung handelt, die bei Raumtemperatur aufbewahrt werden kann, ohne dass es zu einer Abscheidung von Feststoffen kommt, nach einem beliebigen der Ansprüche 1 bis 3.

8. Verfahren nach Anspruch 7, **dadurch gekennzeichnet, dass** es sich bei den Verbindungen (I) und (II), die in der Zusammensetzung vorliegen, um den Alkohol beziehungsweise um das kurzkettige Alkyl(meth)acrylat handelt, welche bei der Umesterungsreaktion eingesetzt werden, und bei der Verbindung (III) um den (Meth)acrylsäureester, der bei dieser Reaktion entsteht.

## Claims

1. Composition in the form of a homogeneous solution which can be stored at ambient temperature without giving rise to precipitation of solid, comprising by weight, the total coming to 100%:
- from 5% to 75% of a dialkyltin oxide, the linear or branched alkyl chain having from 1 to 8 carbon atoms,
- from 10% to 80% of an alcohol R₁OH (I) chosen from butanol, 2-ethylhexanol, decanol, N,N-dimethylaminoethanol, N,N-dimethylaminopropanol, N,N-diethylaminoethanol or tert-butylaminoethanol
- from 10% to 80% of a light alkyl (meth)acrylate (II) chosen from methyl (meth)acrylate or ethyl (meth)acrylate,
- from 0 to 80% of a (meth)acrylic ester (III), butyl (meth)acrylate, 2-ethylhexyl (meth)acrylate dimethylaminoethyl acrylate, dimethylaminopropyl acrylate, diethylaminoethyl acrylate, tert-butylaminoethyl acrylate or dimethylaminoethyl methacrylate.

2. Composition according to Claim 1, comprising:
- from 50% to 70% of a dialkyltin oxide,
- from 10% to 40% of an alcohol R₁OH (I),
- from 10% to 40% of a light alkyl (meth)acrylate (II),
- from 0 to 40% of a (meth)acrylic ester (III).

3. Composition according to Claim 1 or 2, **characterized in that** the dialkyltin oxide is dibutyltin oxide (DBTO).

4. Use of the composition according to any one of the preceding claims as transesterification catalyst for the synthesis of (meth)acrylic esters by transesterification of a light alkyl (meth)acrylate with an alcohol.

5. Use according to Claim 4, **characterized in that** the compounds (I) and (II) present in the composition are respectively the alcohol and the light alkyl (meth)acrylate employed in the transesterification reaction and the compound (III) is the (meth)acrylic ester produced by this reaction.

6. Use according to Claim 5 of a composition comprising by weight, the total coming to 100%:
- from 55% to 65% of dibutyltin oxide,
- from 10% to 20% of N,N-dimethylaminoethanol,
- from 10% to 20% of methyl (meth)acrylate or ethyl (meth)acrylate,
- from 0 to 20% of dimethylaminoethyl (meth)acrylate, as transesterification catalyst for the synthesis of dimethylaminoethyl (meth)acrylate from N,N-dimethyl-aminoethanol and methyl (meth)acrylate or ethyl (meth)acrylate.

7. Process for the continuous synthesis of (meth)acrylic esters of formula (III): 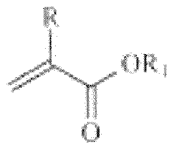 in which R is a hydrogen atom or a methyl group and R₁ is a linear or branched alkyl radical, or a cyclic aliphatic, aryl, alkylaryl or arylalkyl radical, comprising from 4 to 40 carbon atoms, or a linear or branched alkyl radical comprising at least one heteroatom and from 3 to 40 carbon atoms,
by a transesterification reaction of a light alkyl (meth)acrylate of formula (II): 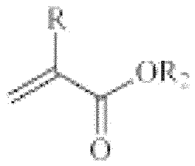 in which R has the abovementioned meanings and R₂ is a linear or branched alkyl chain having from 1 to 4 carbon atoms,
with an alcohol of formula (I):
R₁-OH
in which R₁ has the abovementioned meanings,
in the presence of a transesterification catalyst, **characterized in that** the catalyst is a composition in the form of a homogeneous solution which can be stored at ambient temperature without giving rise to precipitation of solid, according to any one of Claims 1 to 3.

8. Process according to Claim 17, **characterized in that** the compounds (I) and (II) present in the composition are respectively the alcohol and the light alkyl (meth)acrylate employed in the transesterification reaction and the compound (III) is the (meth)acrylic ester produced by this reaction.
